# EUROPEAN PATENT SPECIFICATION

(11) **EP 2 310 488 B1**
(45) Date of publication and mention of the grant of the patent: **19.09.2012**
(21) Application number: 09781466.9
(22) Date of filing: 04.08.2009
(51) Int. Cl.: C12N 1/14

(54) **ADIPOYL-7-ADCA PRODUCING STRAINS**
ADIPOYL-7-ADCA PRODUZIERENDE STÄMME
SOUCHES PRODUISANT DE L'ADIPOYL-7-ADCA

(30) Priority: 05.08.2008 EP 08161831
(43) Date of publication of application: 20.04.2011
(73) Proprietor: DSM Sinochem Pharmaceuticals Netherlands B.V., 2613 AX Delft (NL)
(72) Inventor: KOEKMAN, Bertus, Pieter, NL-2636 BG Schipluiden (NL); BERG, VAN DEN, Marco, Alexander, NL-2685 EH Poeldijk (NL); BOVENBERG, Roelof, Ary, Lans, NL-3062 DA Rotterdam (NL)
(74) Representative: Misset, Onno
(86) International application number: PCT/EP2009/060086
(87) International publication number: WO 2010/015624

(56) References cited:
- WO-A-93/05158
- CRAWFORD L ET AL: "PRODUCTION OF CEPHALOSPORIN INTERMEDIATES BY FEEDING ADIPIC ACID TORECOMBINANT PENICILLIUM CHRYSOGENUM STRAINS EXPRESSING RING EXPANSION ACTIVITY" BIO/TECHNOLOGY, NATURE PUBLISHING CO. NEW YORK, US, vol. 13, 1 January 1995 (1995-01-01), pages 58-62, XP000606289 ISSN: 0733-222X
- GUTIERREZ S ET AL: "EXPRESSION OF THE PENDE GENE OF PENICILLIUM CHRYSOGENUM ENCODING ISOPENICILLIN N ACYLTRANSFERASE IN CEPHALOSPORIUM ACREMONIUM: PRODUCTION OF BENZYLPENICILLIN BY THE TRANSFORMANTS" MOLECULAR AND GENERAL GENETICS, SPRINGER VERLAG, BERLIN, DE, vol. 225, no. 1, 1 January 1991 (1991-01-01), pages 56-64, XP002049910 ISSN: 0026-8925
- ROBIN J ET AL: "Physiological characterisation of Penicillium chrysogenum strains expressing the expandase gene from Streptomyces clavuligerus during batch cultivations. Growth and adipoyl-7-aminodeacetoxycephalosporanic acid production" APPLIED MICROBIOLOGY AND BIOTECHNOLOGY, SPRINGER VERLAG, BERLIN, DE, vol. 57, no. 3, 1 October 2001 (2001-10-01), pages 357-362, XP002482078 ISSN: 0175-7598

## Description

### Field of the invention

The present invention retates to microbial strains capable of producing an N-adipoylated β-lactam compound when cultured in a culture medium comprising Adipic acid

### Background of the invention

Semi-synthetic β-lactam antibiotics (SSA's) are produced on an industrial scale starting from various β-lactam intermediate such as 6-aminopenicillanic acid (6-APA), 7-amino-desacetoxy-cephalosporanic acid (7-ADCA), 7-aminocephalosporanic acid (7-ACA) and 7-amino-3-chloro-3-cephem-carboxylate (7-ACCA); 7-amino-3-[(Z/E)-1-propen-1-yl]-3-cephem-4-carboxylate (7-PACA), 7-aminodeacetylcephalosporanic acid (7-ADAC), 7-amino-3-carbamoyloxymethyl-3-cephem-4-carboxylic acid (7-ACCCA) and others.

The first generation 7-ADCA product was derived from penicillin: G (PenG) whereby both the expansion of the 5-membered penem ring to the 6-membered cephem ring and the subsequent cleavage of the phenylacetic acid side chain of the phenylacetyl-7-ADCA were carried out using chemical reactions. The next generation 7-ADCA product was still obtained starting from PenG but after the chemical ring expansion, the phenylacetic acid side chain of the phenylacetyl-7-ADCA was cleaved off enzymatically using a suitable (penicillin) acylase. Other processes have been developed wherein also the ring expansion of PenG to phenylacetyl-7-ADCA is carried out in vitro using a suitable expandase enzyme, but these processes are of little industrial importance.

The most elegant production process for 7-ADCA comprises the culturing of a *Penicillium chrysogenum*, transformed with and expressing a gene encoding a suitable expandase. This engineered *Penicillium chrysogenum* strain, when grown in the presence of adipic acid as the side chain precursor in the fermentation vessel, produces and excretes adipoyl-7-ADCA - see WO93/05158. and Crawford et al. (Bio/Technol. (1995) 13,58-62) or, for expression in cephalosporium acremonium, Gutierrez et al. (Mol.Gen.Genetics (1991) 225, 56 - 64) In this production process, the adipoyl-7-ADCA is recovered from the fermentation broth, subjected to a suitable acylase to cleave off the adipic acid side chain after which the 7-ADCA thus obtained is further purified, crystallized and dried. Other suitable side chain precursors have been disclosed in WO95/04148 (2-(carboxyethylthio)acetic acid and 3-(carboxymethylthio)-propionic acid), WO95/04149 (2-(carboxyethylthio)propionic acid), WO96/38580 (phenyl acetic acid) and WO98/048034 and WO98/048035 (various dicarboxylic acids). The expandase catalyzes the expansion of the 5-membered ring of the various N-acylated penicillanic acids, thereby yielding the corresponding N-acylated desacetoxycephalosporanic acids.

However, in addition to being used as side chain precursor, adipate may be degraded and used as a carbon source in the primary metabolism of the organism capable of producing the N-adipoyl β-lactam compound. This was for instance demonstrated by Robin et al. (Appl. Microbiol. Biotechnol. (2001) 57, 357-362)) in batch cultures with sucrose as the primary carbon source. After depletion of the glucose and fructose (derived from the sucrose), the formation of adipoyl-6-APA and adipoyl-7-ADCA commenced and in this stage only up to 2% of the adipic acid was incorporated in the β-lactam compounds while the remainder was used as a carbon source. It was suggested by the authors that, due to the similarity between adipic acid and fatty acids, the adipic acid degradation was likely to occur by β-oxidation. In a later study, Thykaer et al. (Metabolic Engineering (2002), 4, 151-158), on the basis of a metabolic network analysis of an adipoyl-7-ADCA producing strain of *Penicillium chrysogenum,* arrived at the conclusion that the adipate degradation takes place in the microbodies (glyoxysomes) by β-oxidation enzymes and not in the cytosol or mitochondria.

The nature of the β-oxidation pathway in (filamentous) fungi and yeast in general, and in *Penicillium chrysogenum* in particular, is still obscure in terms of the intracellular localization, the enzymes involved, as well as the role of the β-oxidation pathway in the total metabolism of the micro-organism. In order to reduce the adipic acid degradation, it has been suggested by Thykaer *et al*. (2002) to delete the enzyme responsible for the adipate degradation, however, without specifying any of such enzymes to be a suitable candidate.

Since adipate is costly compared to glucose or glycerol, adipate degradation during production of the N-adipoyl β-lactam compound is undesirable. Instead, from an industrial production process point of view, an incorporation yield close to 100% is very desirable.

### Detailed description of the invention

The disclosure provides a mutant microbial strain derived from a parent microbial strain capable of producing an N-adipoylated β-lactam compound when cultured in a medium comprising adipic acid characterized in that the mutant microbial strain has an improved incorporation yield of the adipic acid from the culture medium into the N-adipoylated β-lactam compound. The incorporation yield is defined herein as the molar percentage of adipic acid incorporated in the N-adipoylated β-lactam compound relative to the total molar amount of adipic acid consumed. The total molar amount of adipic acid consumed is equal to the total molar amount of adipic acid added to the fermentation process minus the molar amount of adipic acid remaining after the fermentation process. This may be illustrated by the following example: when in a batch fermentation process while culturing a microorganism capable of producing an N-adipoylated β-lactam compound, the initial adipate concentration in the medium is 50 mM and at the end of the fermentation 10 mM of the N-adipoylated β-lactam compound is formed and 15 mM adipate is remaining in the fermentation medium, than the incorporation yield amounts: 10/(50-15)*100 = 29%. Likewise, when 40 mM of the N-adipoylated β-lactam compound is formed and no adipate is remaining in the fermentation medium, the incorporation yield amounts 40/50*100 = 80%.

The improved incorporation yield may be expressed as the relative improvement if the mutant microbial strain compared to the parent microbial strain. For instance, when the parent microbial strain has an incorporation yield of 5% as defined above and the mutant microbial strain of 6%, than the improved incorporation yield of the mutant is 20% (6/5*100 - 100).

Preferably, the mutant strain of the disclosure has an improved incorporation yield of at least 5%, more preferably at least 7.5%, more preferably at least 10%, more preferably at least 15%, more preferably at least 20%, more preferably at least 30%, more preferably at least 40%, more preferably at least 50%, more preferably at least 60%, more preferably at least 70%, more preferably at least 80%, more preferably at least 90%, more preferably at least 95%, more preferably at least 97%, more preferably at least 98%, more preferably at least 99%, more preferably at least 100%,

The maximal improved incorporation yield that can be obtained is dependent on the actual incorporation yield of the parent microbial strain. When the parent microbial strain has an incorporation yield of 5% and 100% is the theoretical maximal incorporation yield, then the mutant microbial strain may have a maximal improved incorporation yield of 1900% (100/5*100 - 100). Likewise, when the parent microbial strain has already an incorporation yield of 50% and 100% is the theoretical maximal incorporation yield, then the mutant microbial strain may have a maximal improved incorporation yield of 100% (100/50*100 - 100).

The N-adipoylated β-lactam compound produced by the microbial strain may be any N-adipoylated β-lactam wherein the β-lactam moiety is a penem or cephem. Preferred N-adipoylated β-lactam compounds are adipoyl-derivates of the intermediates lister before: 6-aminopenicillanic acid (6-APA), 7-amino-desacetoxy-cephalosporanic acid (7-ADCA), 7-aminocephalosporanic acid (7-ACA) and 7-amino-3-chloro-3-cephem-4-carboxylate (7-ACCA), 7-amino-3-[(Z/E)-1-propen-1-yl]-3-cephem-4-carboxylate (7-PACA), 7-aminodeacetylcephalosporanic acid (7-ADAC), 7-amino-3-carbamoyloxymethyl-3-cephem-4-carboxylic acid (7-ACCCA) and others. Most preferred are N-adipoylated cephalosporins, most preferred is adipoyl-7-ADCA.

The parent microbial strain capable of producing an N-adipoylated β-lactam compound may be selected from the group consisting of a fungus, bacterium or yeast. Preferably the microbial strain of the present disclosure is a fungus, more preferably a filamentous fungus. A preferred filamentous fungus may be selected from the group consisting of *Aspergillus, Acremonium, Trichoderma* and *Penicillium.* More preferably the mutant microbial strain of the present disclosure belongs to the species *Penicillium,* most preferably *Penicillium chrysogenum*. A preferred bacterium may be selected from the groups consisting of *Streptomyces, Nocardia,* or *Flavobacterium.*

In a preferred embodiment, the mutant microbial strain of the present disclosure may be derived from a parent microbial strain capable of producing an N-adipoylated β-lactam compound belongs to the species *Penicillium,* most preferably is *Penicillium chrysogenum,* which has been transformed with a gene encoding an expandase, preferably the *Streptomyces clavuligerus cefE* gene, which enables the strain to produce adipoyl-7-ADCA when cultured in the presence of the precursor adipic acid.

In another embodiment, the mutant microbial strain of the present disclosure may be derived from a parent microbial strain capable of producing an N-adipoylated β-lactam compound and belongs to the species *Penicillium,* most preferably is *Penicillium chrysogenum,* and in addition to an expandase gene, preferably the *Streptomyces clavuligerus cefE* gene, has been transformed with a hydroxylase gene, preferably the *Streptomyces clavuligerus cefF* gene, whose expression product converts the 3-methyl side chain of adipoyl-7-ADCA to 3-hydroxymethyl, to give adipoyl-7-aminodeacetylcephalosporanic acid (adipoyl-7-ADAC).

In another embodiment, the mutant microbial strain of the present disclosure may be derived from a parent microbial strain capable of producing an N-adipoylated β-lactam compound and belongs to the species *Penicillium,* most preferably is *Penicillium chrysogenum,* and has been transformed with an expandase/hydroxylase gene, preferably the *Acremonium chrysogenum cefEF* gene, whose expression product converts the 3-methyl side chain of adipoyl-7-ADCA to 3-hydroxymethyl, to give adipoyl-7-aminodeacetylcephalosporanic acid (adipoyl-7-ADAC).

In another embodiment the mutant microbial strain of the present disclosure may be derived from a parent microbial strain capable of producing an N-adipoylated β-lactam compound and belongs to the species *Penicillium,* most preferably is *Penicillium chrysogenum,* and in addition to the genes encoding expandase, preferably the *Streptomyces clavuligerus cefE* gene, and hydroxylase, preferably the *Streptomyces clavuligerus cefF* gene, is further transformed with an acetyltransferase gene, preferably the *Streptomyces clavuligerus cefG* gene, whose expression product (i.e. the acyltransferase) converts the 3-hydroxymethyl side chain to the 3-acetyloxymethyl side chain to give adipoyl-7-ACA.

In a further embodiment the mutant microbial strain of the present disclosure may be derived from a parent microbial strain capable of producing an N-adipoylated β-lactam compound and belongs to the species *Penicillium,* most preferably is *Penicillium chrysogenum* and has been transformed with genes encoding an expandase, preferably the *Streptomyces clavuligerus cefE* gene, a hydroxylase, preferably the *Streptomyces clavuligerus cefF* gene, and an O-carbamoyl transferase enzyme, preferably the *Streptomyces clavuligerus cmcH* gene, resulting in adipoyl-7-amino-3-carbamoyloxymethyl-3-cephem-4-carboxylic acid (7-ACCCA).

Thus, the invention provides a method for the construction and isolation of a mutant microbial strain capable of producing an N-adipoylated, β-lactam compound when cultured in a medium comprising adipic acid with an improved incorporation yield of the adipic acid from the culture medium into the N-adipoylated β-lactam compound. Generally, the method of the invention may comprise the following steps:
a. Obtaining the parent microbial strain in a form which is suitable for the method of the invention. Suitable forms may be conidiospores, vegetative cells (for example, fungal mycelium), protoplasts and others. Obtaining these forms may be carried out according to methods known in the art.
b. Optionally subjecting the population obtained in step a) to mutagenizing conditions in order to increase the frequency of DNA mutations within the population. This step is optional since one may also use the non-mutagenized population obtained in step a) and exploit the natural mutations present in the population and which occur for instance as a result of natural sequence variations. Any suitable mutagenizing condition known in the art may be used, i.e. exposure to suitable mutagens such as far UV-light and chemical mutagens such as nitrosoguanidine. Preferably, the mutagen treatment results in a survival of between 10 and 50% for chemical mutagens and between 0.1 and 1% for physical mutagens like far-UV light.
c. Optionally enriching the population obtained in step a) or in step b) with mutants which have an improved incorporation yield of adipic acid from the culture medium into the N-adipoylated β-lactam compound. Suitable enrichment techniques are:
   - selection using toxic analogues (i.e. fluor-analogs of adipic acid); and/or
   - separation of non-germinated conidiospores (or non-growing cells) from vegetative cells; and/or
   - selective killing of cells for example vegetative fungal cells with pentachlorphenol.
Two or more of these enrichments techniques may also be combined in order to increase the level of enrichment. A preferred method of the invention comprises the following steps:
a. Obtaining conidiospores of a parent microbial strain capable of producing an N-adipoylated β-lactam compound.
b. Optionally subjecting the conidiospores obtained in step a) to mutagenizing conditions
c. Culturing the conidiospores obtained in step a) or step b) in a medium containing adipic acid as the principal carbon source whereby the spores which are able to use adipic acid as a carbon source will germinate, grow and form mycelium
d. Separating the mycelium from the remaining conidiospores
e. Optionally repeating steps b) and c) and d) one or more times
f. Inoculating the conidiospores from step d) on a medium containing a suitable carbon source to allow the growth of single colonies.
g. Replicating the growing colonies from step f) on a medium containing a suitable amount of adipic acid to allow for the identification of non-growing or slower growing colonies.
h. Selecting those colonies which grow in step f) and which belong to the non-growing or slower growing colonies of step g).
Ad step a - Suitable parent microbial strains capable of producing an N-adipoylated β-lactam compound have been listed in the first aspect of the invention. The obtention of conidiospores of the parent microbial strain
Ad step b - Subjecting conidiospores t6 mutagenizing conditions may comprise treatment with a suitable mutagen and may be carried out according to methods known in the art. Suitable mutagens are far UV-light and chemical mutagens such as nitrosoguanidine. Preferably, the mutagen treatment results in a survival of conidiospores between 10 and 50% for chemical mutagens and between 0.1 and 1% for physical mutagens like far-UV light.
Ad step c - The culturing of the conidiospores obtained in step a) or step b) in a medium containing adipic acid as the principal carbon source whereby the spores which are able to use adipic acid as a carbon source will germinate, grow and form mycelium, may be carried out by methods known in the art. Adipic acid may be added to the medium as the acid or in the form of a salt, such as the sodium salt. Suitable concentrations of adipic acid are between 5 and 100 g/l, more preferably 50 - 80 g/l.
Ad step d - The conidiospores that have germinated and have grown to form mycelium may have retained the capacity to use adipic acid as a carbon source. The conidiospores that have not germinated and have not grown to form mycelium may have a reduced or completely lost capacity to use adipic acid as a carbon source. These conidiospores may be separated from the mycelium using methods known in the art. A suitable method is to separate the conidiospores from the mycelium by a suitable filtration technique, such as a Miracloth filter.
Ad step e - Enrichment of conidiospores having a reduced or completely lost capacity to use adipic acid as a carbon source may be carried out by repeating steps a-c, or repeating steps b-c.
Ad step f - Inoculating the conidiospores from step c) on a medium containing a suitable carbon source to allow the growth of single colonies may be carried out on any suitable medium such as a solid medium, e.g. containing agar, according to methods known in the art. Germination of the conidiospores and growth into single colonies may be carried out under conditions (with respect to for instance T, pH, medium composition, carbon sources) which are suitable for the respective conidiospores used in the method of the invention.
Ad step g - Replica plating the colonies obtained in step f with a suitable replicating method (for example, robotic colony picking and re-inoculating) on a medium containing adipic acid as the principal carbon source whereby the colonies which are able to use adipic acid as a carbon source will grow with a certain growth rate and the ones that are not able to use adipic acid as a carbon source will grow slower or not at all. Adipic acid adipic acid as the principal carbon source whereby the colonies which are able to use adipic acid as a carbon source will grow with a certain growth rate and the ones that are not able to use adipic acid as a carbon source will grow slower or not at all. Adipic acid may be added to the medium as the acid or in the form of a salt, such as the -sodium salt. Suitable concentrations of adipic acid are between 5 and 100 g/l, more preferably 50-80 g/l.
Ad. step h - Selecting those colonies which grow in step f) and which belong to the non-growing or slower growing colonies of step g).

The mutant microbial strains selected in step h may be tested for their incorporation of adipic acid in the N-adipoylated β-lactam compound when cultured in a medium comprising adipic acid. Only those mutant microbial strains are selected which have an improved incorporation yield of at least 30%, more preferably at least 40%, more preferably at least 50%, more preferably at least 60%, more preferably at least 70%, more preferably at least 80%, more preferably at least 90%, more preferably at least 95%, more preferably at least 97%, more preferably at least 98%, more preferably at least 99%, most preferably: of 100%, whereby the incorporation yield is as defined hereinbefore.

The mutant microbial strains selected in step h may optionally be tested for their reduced or completely lost capacity to use adipic acid as a carbon source by measuring their growth in a suitable medium with either adipic acid or one or more sugars as the sole carbon source.

### MATERIALS AND METHODS

Adipoyl-7-ADCA concentrations were determined using HPLC as described in detail in WO98/48035.

### EXAMPLES

### Example 1

*chrysogenum* deposited on 2 June 1995 at the Centraal Bureau voor Schimmelcultures under number CBS 455.95 was transformed with the *cefE* gene encoding the expandase from *Streptomyces clavuligerus* as described in detail in WO93/05158.

For the selection of a growth medium for the detection of adipoyl-7-ADCA producing *Penicillium chrysogenum* mutant strains with an improved incorporation of adipic acid from the medium into adipoyl-7ADCA, spores of the adipoyl-7-ADCA producing *Penicillium chrysogenum* parent strain were inoculated on agar plates containing a medium as disclosed in example 2 of WO98/373179 having the following composition (in g/l): urea, 4.5; (NH₄)₂SO₄, 1.1; Na₂SO₄, 2.9; KH₂PO₄, 5.2; K₂HPO₄.3H₂O, 4.8 and 10 ml/l of a trace elements solution (containing in g/l: citric acid.H₂O, 150; FeSO₄.7H₂O, 15; MgSO₄.7H₂O, 150; H₃BO₃, 0.0075; CuSO₄.5H₂O, 0.24; CoSO₄.7H₂O, 0.375; ZnSO₄.7H₂O, 1.5; MnSO₄.H₂O, 2.28; CaCl₂.2H₂O, 0.99) - pH before sterilization 6.5.

The medium was supplemented with glucose and/or adipic acid as carbon source as indicated in Table 1. The agar plates were incubated at 25°C and after 7 days of growth, the colonies were inspected visually.

**Table 1.**

| Medium | Glucose (g/L) | Adipic acid (g/L) | growth | Description |
|---|---|---|---|---|
| 1 | 0.0 | 10 | +/- | hardly if any growth |
| 2 | 0.0 | 80 | +/- | hardly if any growth |
| 3 | 0.1 | 0 | + | Small colonies |
| 4 | 0.1 | 10 | ++ | Medium colonies |
| 5 | 0.1 | 80 | +++ | Large colonies |

From the table can be concluded that the adipoyl-7-ADCA producing *Penicillium chrysogenum* strain hardly grows on an agar medium with adipic acid as the sole carbon source. However, in the presence of a small amount of glucose, growth is enhanced substantially in the presence of adipic acid, thus enabling the discrimination between adipate utilizing strains and adipate non-utilizing strains.

### Example 2

### Selection of adipoyl-7-ADCA producing Penicillium chrysogenum mutant strains with an improved incorporation of adipic acid from the medium into adipoyl-ADCA

A suspension of conidiospores of the adipoyl-7-ADCA producing *Penicillium chrysogenum* strain constructed as described in Example 1 was irradiated with UV-light to a survival of 0.2%. Subsequently, shake flasks containing 25 ml of a liquid medium described in Example 1 supplemented with 40 g/L adipic acid as the sole carbon source were inoculated with the UV-irradiated suspension of conidiospores and incubated for 2 days at 25°C and at 280 rpm until sufficient germination took place. Spores that were not able to germinate were suspected to have lost (most of) their ability to use adipic acid as a carbon source. After 95% of the spores were germinated, the mycelium and remaining spores were separated by passing the fermentation broth through a Miracloth filter.

The spores passing the filter were inoculated in fresh liquid medium containing 40 g/L adipic acid as the sole carbon source. After 3 days culturing, the mycelium and remaining spores were again separated by passing the fermentation broth through a Miracloth filter and the spores thus collected were inoculated on the agar medium as described in Example 1 containing 80 g/l lactose monohydrate and 5 g/l glucose monohydrate as carbon sources. After growth of the colonies, circa 25,000 colonies were replica plated onto fresh agar plates with the composition of medium nr 5 of Table 1.

Of the non-growing colonies, 125 strains were selected and tested in the liquid medium as described in the above paragraph, supplemented with 20 g/l adipic acid as the side chain precursor. After seven days the cultures were sampled and the supernatant was used to determine the adipoyl-7-ADCA level (as described in US6410259). Table 2 shows that 2 strains were obtained which had a significantly improved incorporation of adipic acid from the medium into adipoyl-7-ADCA compared to the control (parent strain).

**Table 2.**

| **Strain** | **Adipate incorporation Yield* (%)** | **Improvement factor of the adipate incorporation yield** | **Adipoyl-7-ADCA productivity (control=100%)** |
|---|---|---|---|
| Control (parent) | 22.4 | 1.00 | 100 |
| Mutant A | 30.4 | 1.36 | 110 |
| Mutant B | 33.6 | 1.50 | 120 |
| Mutant C | 38.8 | 1.70 | 62 |
| Mutant D | 89.4 | 4.00 | 50 |

| | | | |
|---|---|---|---|
| * see definition in the text | | | |

## Claims

1. A method for the construction of a mutant microbial strain capable of producing an N-adipoylated β-lactam compound when cultured in a culture medium comprising adipic acid and having an improved incorporation yield of the adipic acid from the culture medium into the N-adipoylated β-lactam compound compared to the non-mutant parent strain, the method comprising the steps of:
a) Obtaining conidiospores of a parent microbial strain capable of producing an N-adipoylated β-lactam compound
b) Subjecting the conidiospores obtained in step a) to mutagenizing conditions
c) Culturing the conidiospores obtained in step b) in a medium containing adipic acid as the principal carbon source whereby the spores which are able to use adipic acid as a carbon source will germinate, grow and form mycelium
d) Separating the mycelium from the remaining conidiospores
e) Optionally repeating steps b) and c) and d) one or more times
f) Inoculating the conidiospores from step d) on a medium containing a suitable carbon source to allow the growth of single colonies.
g) Replicating the growing colonies from step f) on a medium containing a suitable amount of adipic acid to allow for the identification of non-growing or slower growing colonies.
h) Selecting those colonies which grow in step f) and which belong to the non-growing or slower growing colonies of step g).

2. The method of claim 1 **characterized in that** the improved incorporation yield is at least 5% wherein said improved incorporation yield is expressed as the relative improvement of the mutant microbial strain compared to the parent microbial strain.

3. The method of claim 1 or 2 wherein the N-adipoylated β-lactam compound is selected from the group consisting of adipoyl-6-APA, adipoyl-7-ADCA, adipoyl-7-ACA, adipoyl-7-ACCA, adipoyl-7-PACA, adipoyl-7-ADAC, adipoyl-7-ACCCA.

4. The method of claims 1-3 wherein the strain is a fungus or bacterium.

5. The method of claim 4 wherein the fungus belongs to the genus *Penicillium.*

6. The method of claim 5 wherein the fungus is *Penicillium chrysogenum*, preferably transformed with and expressing a gene encoding an expandase or an expandase/hydroxylase.

## Patentansprüche

1. Verfahren zum Konstruieren eines mutierten Mikroorganismenstamms, der fähig ist, wenn er in einem Kulturmedium, das Adipinsäure umfasst, kultiviert wird, eine N-adipoylierte β-Lactamverbindung zu produzieren und der im Vergleich zu dem nichtmutierten Elternstamm eine verbesserte Adipinsäureeinbaurate aus dem Kulturmedium in die N-adipoylierte β-Lactamverbindung aufweist, wobei das Verfahren die folgenden Schritte umfasst:
a) Gewinnen von Konidiosporen eines Mikroorganismenelternstamms, der fähig ist, eine N-adipoylierte β-Lactamverbindung zu produzieren,
b) Aussetzen der in Schritt a) gewonnenen Konidiosporen an mutagenisierende Bedingungen,
c) Kultivieren der in Schritt b) gewonnenen Konidiosporen in einem Medium, das Adipinsäure als Hauptkohlenstoffquelle enthält, wodurch die Sporen, die fähig sind, Adipinsäure als Kohlenstoffquelle zu nutzen, keimen, wachsen und ein Myzel bilden werden,
d) Abtrennen des Myzels von den verbleibenden Konidiosporen,
e) gegebenenfalls ein- oder mehrmaliges Wiederholen der Schritte b) und c) und d),
f) Inokulieren der Konidiosporen aus Schritt d) auf ein Medium, das eine geeignete Kohlenstoffquelle enthält, um das Wachstum von Einzelkolonien zu gestatten,
g) Replizieren der wachsenden Kolonien aus Schritt f) auf einem Medium, das eine geeignete Menge Adipinsäure enthält, um die Identifikation von nicht oder langsamer wachsenden Kolonien zu gestatten,
h) Selektieren von denjenigen Kolonien, die in Schritt f) wachsen und die zu den nicht oder langsamer wachsenden Kolonien von Schritt g) gehören.

2. Verfahren nach Anspruch 1, **dadurch gekennzeichnet, dass** es sich bei der verbesserten Einbaurate um mindestens 5% handelt, wobei die verbesserte Einbaurate als relative Verbesserung des mutierten Mikroorganismenstamms im Vergleich zu dem Elternmikroorganismenstamm ausgedrückt wird.

3. Verfahren nach Anspruch 1 oder 2, wobei die N-adipoylierte β-Lactamverbindung aus der Gruppe Adipoyl-6-APA, Adipoyl-7-ADCA, Adipoyl-7-ACA, Adipoyl-7-ACCA, Adipoyl-7-PACA, Adipoyl-7-ADAC-Adipoyl-7-ACCCA stammt.

4. Verfahren nach den Ansprüchen 1-3, wobei es sich bei dem Stamm um einen Pilz oder ein Bakterium handelt.

5. Verfahren nach Anspruch 4, wobei der Pilz zu der Gattung *Penicillium* gehört.

6. Verfahren nach Anspruch 5, wobei es sich bei dem Pilz um *Penicillium chrysogenum* handelt, der vorzugsweise mit einem Gen, das für eine Expandase oder eine Expandase/Hydroxylase codiert, transformiert ist und dieses exprimiert.

## Revendications

1. Procédé pour la construction d'une souche microbienne mutante capable de produire un composé β-lactame N-adipoylé lorsqu'elle est cultivée dans un milieu de culture comprenant de l'acide adipique et ayant un rendement d'incorporation amélioré de l'acide adipique à partir du milieu de culture dans le composé β-lactame N-adipoylé comparé à la souche parente non mutante, le procédé comprenant les étapes consistant à :
a) obtenir des conidiospores d'une souche microbienne parente capable de produire un composé β-lactame N-adipoylé
b) soumettre les conidiospores obtenues dans l'étape a) à des conditions de mutagenèse
c) cultiver les conidiospores obtenues dans l'étape b) dans un milieu contenant de l'acide adipique en tant que source principale de carbone de telle manière que les spores qui sont capables d'utiliser l'acide adipique germent, croissent et forment un mycélium
d) séparer le mycélium des conidiospores restantes
e) facultativement répéter les étapes b) et c) et d) une ou plusieurs fois
f) inoculer les conidiospores de l'étape d) sur un milieu contenant une source de carbone adaptée pour permettre la croissance de colonies individuelles,
g) répliquer les colonies croissantes de l'étape f) sur un milieu contenant une quantité adaptée d'acide adipique pour permettre l'identification de colonies non croissantes ou croissant plus lentement.
h) sélectionner les colonies qui croissent dans l'étape f) et qui appartiennent aux colonies non croissantes ou croissant plus lentement de l'étape g).

2. Procédé de la revendication 1 **caractérisé en ce que** le rendement d'incorporation amélioré est au moins 5 %, ledit rendement d'incorporation amélioré étant exprimé comme étant l'amélioration relative de la souche microbienne mutante comparée à la souche microbienne parente.

3. Procédé de la revendication 1 ou 2 dans lequel le composé β-lactame N-adipoylé est choisi dans le groupe constitué d'adipoyl-6-APA, adipoyl-7-ADCA, adipoyl-7-ACA, adipoyl-7-ACCA, adipoyl-7-PACA, adipoyl-7-ADAC, adipoyl-7-ACCCA.

4. Procédé des revendications 1 à 3 dans lequel la souche est un champignon ou une bactérie.

5. Procédé de la revendication 4 dans lequel le champignon appartient au genre *Penicillium.*

6. Procédé de la revendication 5 dans lequel le champignon est *Penicillium chrysogenum,* de préférence transformé avec et exprimant un gène codant pour une expandase ou une expandase/hydroxylase.
